Europäisches Patentamt

(19) European Patent Office

Office européen des brevets

(11) **EP 0 661 930 B1**

(12) **FASCICULE DE BREVET EUROPEEN**

(45) Date de publication et mention
de la délivrance du brevet:
**16.09.1998 Bulletin 1998/38**

(21) Numéro de dépôt: 94922300.2

(22) Date de dépôt: **22.07.1994**

(51) Int. Cl.$^6$: **A23L 1/09**, A23L 1/236,
A61K 7/06, A61K 7/16,
A61K 7/48, A61K 31/715,
C07C 31/18

(86) Numéro de dépôt international:
**PCT/FR94/00927**

(87) Numéro de publication internationale:
**WO 95/02967 (02.02.1995 Gazette 1995/06)**

(54) **COMPOSITIONS LIQUIDES VISQUEUSES DE XYLITOL ET LEUR PROCEDE DE PREPARATION**

Viskose Xytolzusammensetzungen und ihre Herstellung

VISCOUS LIQUID XYLITOL COMPOSITIONS AND METHOD FOR PREPARING SAME

(84) Etats contractants désignés:
**AT BE CH DE DK ES GB GR IT LI LU NL SE**

(30) Priorité: **26.07.1993 FR 9309180**

(43) Date de publication de la demande:
**12.07.1995 Bulletin 1995/28**

(73) Titulaire: **Roquette Frères
62136 Lestrem (FR)**

(72) Inventeurs:
• **DUFLOT, Pierrick
F-62136 Richebourg (FR)**

• **CABOCHE, Jean-Jacques
F-62400 Béthune (FR)**

(74) Mandataire: **Boulinguiez, Didier
Cabinet Plasseraud
84, rue d'Amsterdam
75440 Paris Cedex 09 (FR)**

(56) Documents cités:
**EP-A- 0 421 882          EP-A- 0 431 995
WO-A-92/06943**

## Description

La présente invention concerne des compositions liquides visqueuses de xylitol ainsi qu'un procédé pour leur préparation.

De façon plus précise, elle concerne de nouvelles compositions liquides visqueuses et difficilement cristallisables de xylitol contenant du D-arabitol et des oligomères ou polymères non réducteurs du glucose. L'invention concerne aussi un procédé permettant d'obtenir de telles compositions visqueuses et difficilement cristallisables.

Les sirops épais ou visqueux obtenus par hydrolyse puis hydrogénation de l'amidon et notamment les sirops de sorbitol ont trouvé une large application en tant que succédanés des sirops de saccharose dans de nombreuses applications alimentaires ou pharmaceutiques. Bien qu'il soit moins sucré que le sucre, le sorbitol possède en effet sur celui-ci l'avantage de ne pas être cariogène. Les faibles effets viscosifiants du sorbitol, liés à son bas poids moléculaire, peuvent en effet être compensés par une concentration plus élevée des sirops qui sont alors tout aussi visqueux et incristallisables que les sirops de saccharose.

Les propriétés humectantes intrinsèques des polyols ont aussi permis aux plus solubles d'entre eux de trouver des applications dans le domaine de la cosmétique. La grande solubilité du sorbitol et son pouvoir élevé de rétention d'eau a ainsi permis aux sirops concentrés de sorbitol de s'octroyer une place de choix en tant qu'excipient des pâtes dentifrices par exemple.

Le xylitol, autre polyol, possède les avantages d'être pratiquement aussi sucré que le saccharose et d'être de plus, tout comme le sorbitol, non cariogène.

Cependant, son bas poids moléculaire, plus faible encore que celui du sorbitol et sa faible solubilité, inférieure au dessous de 30°C à celles du sorbitol et du saccharose ne lui permettent pas à lui seul de former des sirops suffisamment épais qui ne cristallisent pas et qui puissent remplacer les sirops de saccharose ou de sorbitol dans les applications alimentaires ou pharmaceutiques ou bien les sirops de sorbitol ou de glycérol dans les applications cosmétiques.

Des compositions concentrées liquides visqueuses de xylitol ont pourtant déjà été décrites, par exemple dans la demande européenne de brevet n° 431.995. Elles sont visqueuses et incristallisables mais contiennent au maximum 50 % de xylitol, le reste de la composition étant essentiellement constitué par un hydrolysat d'amidon hydrogéné riche en maltitol.

D'autres compositions concentrées liquides de xylitol, contenant de 50 % à 90 % de xylitol, 10 % à 50 % d'autres polyols et présentant un certain retard à la cristallisation ont aussi été décrites dans la demande internationale de brevet WO92/06943. Par retard à la cristallisation, on entend le cas où le xylitol cristallise lentement et difficilement sous forme de microcristaux à peine palpables ou discernables dans les compositions liquides concentrées qui les contiennent. Ces compositions concentrées liquides ne sont toutefois réellement incristallisables à 20°C que si leur richesse en xylitol est moindre que 70 % et leur concentration, inférieure à 70 % de matières sèches. Dans ces conditions particulières, ces compositions sont très peu visqueuses. De plus, il n'est pas étonnant que le xylitol n'y cristallise pas puisque celui-ci ne semble pas alors se trouver au dessous de sa limite de solubilité. Dans ces compositions plus riches en xylitol ou plus concentrées, le retard à la cristallisation du xylitol est causé par la présence d'autres polyols de faible poids moléculaire tels que le sorbitol, maltitol, mannitol et glycérol. Il est aussi renforcé éventuellement par d'autres polyols présents dans les eaux mères de la cristallisation du xylitol obtenu par hydrogénation d'hydrolysats de bois ou de rafles de maïs. Ces polyols présents dans les eaux-mères possèdent eux aussi un faible poids moléculaire. Il s'agit notamment de L-arabitol et de galactitol. On les rencontre toujours dans les compositions concentrées liquides de xylitol lorsque ces eaux mères sont employées comme source de xylitol en lieu et place de solutions formées par dissolution de xylitol cristallisé pur et addition de sorbitol, mannitol, maltitol, glycérol.

Les procédés employés pour préparer ces compositions concentrées liquides de xylitol consistent dans tous les cas à additionner à des solutions de xylitol brutes telles que des eaux-mères de cristallisation ou reformées à partir de xylitol cristallisé, des polyols de faible poids moléculaire tels que le sorbitol, mannitol, maltitol ou hydrolysats d'amidon hydrogénés à haute teneur en maltitol.

L'utilisation d'eaux-mères provenant de l'hydrogénation et de la cristallisation d'hydrolysats de bois ou de rafles de maïs est certes avantageuse sur un plan économique lorsqu'il s'agit de préparer des compositions liquides concentrées de xylitol. Elle permet en effet d'écouler un sous produit mais se trouve cependant restreinte dans les applications alimentaires, pharmaceutiques ou cosmétiques parce que le L-arabitol présent dans ces hydrolysats n'est pas un polyol de la série naturelle et que le galactitol qui y est également présent est connu pour causer la cataracte.

L'obtention de compositions à viscosité améliorée ou de compositions concentrées liquides de xylitol, incristallisables ou présentant un certain retard à la cristallisation et qui sont exemptes de L-arabitol et de galactitol n'a donc jusqu'à maintenant pu être réalisée que par la mise en oeuvre de xylitol cristallisé pur. Ces compositions sont donc onéreuses à produire puisque le xylitol cristallisé qu'il faut mettre en oeuvre pour leur obtention est en lui-même un produit onéreux. Par ailleurs, le sorbitol, le mannitol, le maltitol, le glycérol et les hydrolysats d'amidon hydrogénés à haute teneur en maltitol sont aussi des produits onéreux. De plus ces produits ne permettent pas d'atteindre des niveaux de viscosité souhaitables.

Or, il existe un besoin de compositions visqueuses ou de compositions concentrées liquides de xylitol qui soient incristallisables ou qui présentent un certain retard à la cristallisation, qui ne présentent pas de danger pour la santé et qui soient peu onéreuses à obtenir.

De telles compositions peuvent avantageusement trouver application dans la confection d'excipients pour pâtes dentifrices, produits cosmétiques ou pharmaceutiques et dans la fabrication de confiseries diverses et variées.

Il est connu par le brevet européen n° 421.882 dont la demanderesse est titulaire d'obtenir des sirops de xylitol ayant une teneur en xylitol de 80 % à 90 %, le reste étant essentiellement constitué par le D-arabitol, pentitol de la série D naturelle des sucres. Ces sirops peu visqueux et particulièrement bien adaptés à la cristallisation du xylitol, sont obtenus par transformation microbiologique du glucose en D-arabitol, oxydation microbiologique du D-arabitol en D-xylulose, isomérisation enzymatique du D-xylulose en D-xylose puis hydrogénation catalytique du mélange de D-xylulose et de D-xylose en xylitol et D-arabitol. Ces sirops de xylitol qui ne contiennent pas de L-arabitol ni de galactitol mais uniquement du D-arabitol à titre de polyol accompagnant, permettent l'extraction aisée de trois jets de cristaux de xylitol pur.

La concentration de D-arabitol atteint alors jusqu'à 50 % et davantage de la matière sèche des eaux mères, prouvant à l'évidence que ce D-arabitol n'est pas un anti-cristallisant du xylitol.

Il est donc du mérite de la demanderesse d'avoir constaté que l' association au D-arabitol présent dans ces sirops, d'oligomères ou de polymères de hauts poids moléculaires non réducteurs du glucose, permettait d'y entraver la cristallisation du xylitol. Cette constatation est d'autant plus surprenante que cette association n'est pas dépendante de la présence dans ces sirops de quantités notoires des anticristallisants prétendus du xylitol que sont le sorbitol, le maltitol, le mannitol, le glycérol, le L-arabitol ou le galactitol, anticristallisants qui sont tous de bas poids moléculaire.

En outre, la présence dans ces sirops de xylitol d'oligomères ou de polymères non réducteurs du glucose de hauts poids moléculaires permet de conférer aux sirops mêmes dilués, un certain corps, utile dans de nombreuses applications.

Par oligomères ou polymères de hauts poids moléculaires non réducteurs du glucose, on entend : le maltotriitol, le maltotétraitol et leurs homologues supérieurs qui sont obtenus par hydrolyse incomplète de l'amidon puis hydrogénation de ces hydrolysats ainsi que les isomères de ces composés dont les molécules sont ramifiées.

Les compositions liquides visqueuses de xylitol selon l'invention sont donc caractérisées par le fait qu'elles contiennent :

- de 51 % à 80 % de xylitol
- de 0,1 % à 44 % de D-arabitol
- de 5 % à 48,9 % d'oligomères ou de polymères non réducteurs du glucose.

De façon préférée, elles contiennent :

- de 53 % à 75 % de xylitol
- de 2 % à 35 % de D-arabitol
- de 8 % à 45 % d'oligomères ou polymères non réducteurs du glucose.

De façon encore plus préférentielle, elle contiennent :

- de 55 % à 75 % de xylitol
- de 4 % à 30 % de D-arabitol
- de 10 % à 41 % d'oligomères ou polymères non réducteurs du glucose.

La société demanderesse a par ailleurs remarqué que la limite de cristallisation ou de solubilité du xylitol pur dans l'eau en fonction de la température pouvait être estimée par l'équation :

$$y = \frac{83,2x + 9130}{83 - x}$$

où x représente la température de la solution de xylitol en degrés centigrades et où y représente la limite de solubilité du xylitol en grammes de xylitol pour 100 grammes d'eau.

Les données calculées à partir de cette équation sont en excellent accord avec les valeurs mesurées de la solubilité du xylitol dans l'eau en fonction de la température telles qu'on peut les trouver par exemple en page 368 de l'ouvrage : "Le sucre, les sucres, les édulcorants et les glucides de charge dans les I.A.A." - collection Sciences et techniques agro-alimentaires, J.L. Multon coordinateur, Tec et Doc, Lavoisier APRIA 1992.

C'est ainsi qu'aux températures de 10°, 20°, 30°, 40°, 50° et 60°C sont associées des solubilités mesurées du xylitol de respectivement 138, 168, 217, 292, 400 et 614 g/100 cm$^3$ d'eau. Il se fait que l'équation sus-indiquée permet de calculer les valeurs suivantes pour les mêmes températures : 136,5, 171, 219, 290, 402 et 614 démontrant par là même l'excellente corrélation des valeurs théoriques et expérimentales dans l'intervalle de température allant de 0° à 70°C environ.

L'arc d'hyperbole représentant cette équation sépare dans l'intervalle où elle a un sens, le plan en une zone sous la courbe où le xylitol est à l'état de sous saturation et ne peut donc pas cristalliser et une zone de sursaturation, située au dessus de la courbe et où le xylitol doit normalement cristalliser.

Il se trouve cependant, ce que la demanderesse a constaté après de nombreux essais, que la présence simultanée dans les solutions de xylitol, de D-arabitol et d'oligomères ou de polymères non réducteurs du glucose, permettait aussi de façon inexpliquée, d'augmenter la solubilité du xylitol ou de retarder fortement sa cristallisation. C'est ainsi que les compositions de l'invention permettent d'obtenir des caractéristiques de cristallisation retardée pour des taux de sursaturation du xylitol compris entre 1,1 et 1,2 et des compositions incristallisables pour des taux de sursaturation compris entre 1,0 et 1,1. Pour de tels taux, les concentrations limites en xylitol des compositions selon l'invention s'obtiennent en multipliant par 1,1 ou 1,2 les numérateurs de l'équation précédente.

Il s'ensuit que des compositions liquides de xylitol préférées et présentant des caractéristiques de cristallisation retardée ou qui sont incristallisables à températures données sont caractérisées par le fait que leur teneur en eau est ajustée de manière à ce que leur teneur en xylitol, exprimée en grammes de xylitol pour 100 grammes d'eau soit comprise entre les valeurs définies par les équations :

$$\frac{83,2x + 9130}{83 - x} \text{ et } \frac{100x + 11000}{83 - x}$$

(x représentant la température en degrés centigrades des compositions).

D'autres compositions liquides concentrées de xylitol encore plus préférées et qui sont incristallisables à températures données sont caractérisées par le fait que leur teneur en eau est ajustée de manière à ce que leur teneur en xylitol soit comprise entre les valeurs définies par les équations :

$$\frac{83,2x + 9130}{83 - x} \text{ et } \frac{91,5x + 10050}{83 - x}$$

(x représentant la température en degrés centigrades des compositions).

Lorsque l'on désire que les compositions liquides de xylitol selon l'invention présentent des caractéristiques de cristallisation retardée à environ 20°C, il faut que leur teneur en eau soit ajustée de manière à ce que leur teneur en xylitol, exprimée en grammes de xylitol pour 100 grammes d'eau soit comprise entre 188 g et 206 g.

Lorsque l'on désire que ces mêmes compositions soient incristallisables à environ de 20°C, il faut que leur teneur en eau soit ajustée de manière à ce que leur teneur en xylitol, exprimée en grammes de xylitol pour 100 grammes d'eau, soit comprise entre 171 g et 188 g. Un procédé d'obtention des compositions liquides de xylitol selon l'invention, consiste à mélanger aux sirops de xylitol obtenus selon le brevet européen n° 421.882, des oligomères ou polymères non réducteurs du glucose. Ceux-ci sont de préférence de hauts poids moléculaires et proviennent préférentiellement de dextrines ou de maltodextrines hydrogénées.

Un second procédé permettant la préparation des compositions liquides de xylitol selon l'invention, consiste à utiliser des sirops de xylose ou des poudres de xylose obtenus selon le procédé fermentaire décrit dans le brevet européen n°421.882, à ajouter des oligomères ou des polymères réducteurs provenant de dextrines, de maltodextrines ou d'hydrolysats d'amidon pauvres en glucose et en maltose et de préférence dépourvus de ces deux sucres et enfin à procéder à une hydrogénation du mélange obtenu dans des conditions connues de l'homme du métier. Dans ce cas les quantités d'oligomères et de polymères réducteurs ajoutés seront choisies de façon à ce que la composition liquide obtenue contienne au moins 51 % de xylitol. Il est à noter que la composition liquide obtenue en appliquant ce procédé sera alors conforme à l'invention car elle contiendra obligatoirement du D-arabitol en raison de la présence plus ou moins grande de D-xylulose dans le xylose fermentaire utilisé.

Un troisième procédé d'obtention des compositions liquides de xylitol selon l'invention consiste à procéder aux étapes déjà décrites dans le brevet européen n° 421.882, de la transformation microbiologique et chimique du glucose en xylitol mais en effectuant tout ou partie de la transformation fermentaire ou enzymatique en présence d'oligomères ou de polymères du glucose.

Par oligomères ou polymères du glucose, on entend : le maltotriose, le maltotétraose et leurs homologues supérieurs qui sont obtenus par hydrolyse incomplète de l'amidon ainsi que les isomères de ces composés dont les molécules sont ramifiées.

De façon surprenante, la société demanderesse a observé que la présence de ces oligomères ou polymères réducteurs du glucose à côté du glucose n'entravait nullement les transformations microbiologiques du glucose en D-arabitol puis du D-arabitol en D-xylulose, que ces mêmes oligomères ou polymères demeuraient inaltérés au cours de ces transformations microbiologiques et qu'ils ne gênaient pas davantage la réaction d'isomérisation enzymatique du D-xylulose en D-xylose. L'hydrogénation catalytique du sirop obtenu et contenant ces oligomères et polymères de glucose conduit alors à un sirop contenant du xylitol (provenant de la réduction du D-xylose et pour moitié de celle du D-xylulose), du D-arabitol (provenant pour moitié de la réduction du D-xylulose) et des oligomères ou polymères non réducteurs du glucose (provenant de leurs équivalents réducteurs).

Ce troisième procédé d'obtention des compositions liquides de xylitol selon l'invention, consiste donc à procéder aux transformations microbiologiques du glucose en D-arabitol puis D-xylulose, à l'isomérisation enzymatique du D-xylulose en un mélange de D-xylose et de D-xylulose puis à l'hydrogénation catalytique de ce mélange, caractérisé en ce que les transformations microbiologiques, l'isomérisation et l'hydrogénation ont lieu en présence d'oligomères et/ou de polymères du glucose.

Ce procédé est d'autant plus préféré que l'on peut avoir recours dans ce cas à une matière première unique, vraiment peu onéreuse et qui peut être constituée par les eaux mères de la cristallisation du dextrose par exemple, eaux mères qui contiennent environ 4 % à 7 % d'oligomères ou polymères de glucose qui ont échappé aux saccharifications enzymatiques.

Une autre matière première utilisable dans le procédé de l'invention peut être un hydrolysat brut d'amidon obtenu par liquéfaction acide puis saccharification enzymatique. Un tel hydrolysat est moins onéreux à produire qu'un hydrolysat enzymatique double possédant une très haute teneur en glucose. En outre la liquéfaction acide employée en place de la liquéfaction enzymatique , forme des produits de réversion et des molécules ramifiées qui échappent à la saccharification enzymatique ultérieure et demeurent ainsi sous forme d'oligomères ou de polymères de hauts poids moléculaires, inhibiteurs de la cristallisation du xylitol et conférant une certaine viscosité aux compositions.

Pour mettre en oeuvre le procédé selon l'invention lorsqu'on utilise un hydrolysat brut d'amidon obtenu par liquéfaction acide comme matière première, on conduit habituellement cette liquéfaction en portant à une température d'environ 130° à 150°C un lait d'amidon d'une concentration de 25 % à 45 % en présence d'une quantité d'acide chlorhydrique suffisante pour obtenir un pH de 1,5 à 2,0.

On préfère conduire cette liquéfaction jusqu'à l'obtention d'un DE (Dextrose Equivalent) de 30 à 50.

On procède ensuite sur cet hydrolysat brut obtenu par voie acide, à une saccharification enzymatique à l'aide d'une amyloglucosidase. Cette saccharification est habituellement conduite à une température de 50° à 60°C durant 20 à 200 heures, à un pH compris entre 5,0 et 6,0 et à une matière sèche comprise entre 20 % et 40 % à l'aide de 4.000 à 500.000 unités internationales d'activité enzymatique par kilogramme de substrat sec. N'importe quelle amyloglucosidase peut être utilisée mais on préfère cependant employer les amyloglucosidases fongiques. On conduit de préférence la saccharification jusqu'à son terme c'est à dire jusqu'à l'obtention d'une teneur en glucose vrai de 75 % à 90 %. La teneur de ces sirops en oligomères ou polymères de glucose est alors généralement comprise entre 5 % et 20 %.

Une autre matière première encore et qui est préférée, est constituée par un hydrolysat enzymatique de dextrine. Dans le cadre de la présente invention, on entend par le terme dextrines les produits obtenus par chauffage de l'amidon amené à un faible taux d'humidité en présence généralement de catalyseurs acides ou basiques. Ce "grillage à sec" de l'amidon, le plus couramment en présence d'acide entraîne à la fois une dépolymérisation de l'amidon et une condensation des fragments d'amidon ainsi obtenus ; il conduit à l'obtention de molécules très ramifiées et de hauts poids moléculaires qui ne sont plus complètement hydrolysables par les amyloglucosidases.

Lorsque l'on utilise des dextrines en tant que matières premières dans le procédé selon l'invention, on préfère utiliser des dextrines obtenues par grillage à sec de l'amidon en présence d'un catalyseur acide comme l'acide chlorhydrique. L'acide est par exemple pulvérisé sur l'amidon et le mélange obtenu est préséché de 80° à 130°C jusqu'à une teneur en eau inférieure ou égale à 5 %. Le mélange est ensuite grillé à une température d'environ 140° à 250°C pendant une durée de 30 minutes à environ 6 heures pour obtenir la dextrine qui présente en fin de réaction un DE de 0,5 à 10 environ. On peut utiliser pour la préparation de ces dextrines n'importe quel type d'amidon, et notamment l'amidon de mais, de blé, de riz ou les fécules de pommes de terre, de manioc.

Traditionnellement, les dextrines sont classées en deux catégories : les dextrines blanches dont l'aspect est peu différent de celui de matière première utilisée, et les dextrines jaunes, produites dans des conditions plus énergiques et dont l'intensité de coloration peut être corrélée au degré de modification de la structure native. Les quatre types de réaction intervenant lors de la dextrinification sont aux faibles températures essentiellement l'hydrolyse des liaisons alpha 1-4 puis, aux températures plus élevées des réactions de condensation, de transglycosylation et enfin d'anhydrisation interne.

Dans le procédé préféré de la présente invention, on aime mieux mettre en oeuvre les dextrines blanches qui sont pratiquement exemptes d'anhydrides internes du glucose. En outre, l'hydrolyse enzymatique de dextrines plus transformées ne permettrait plus d'atteindre des richesses de glucose qui soient suffisantes pour obtenir un taux de xylitol supérieur à 51 %.

Des dextrines telles que celles commercialisées par la société demanderesse sous les marques TACKIDEX$^R$ 135, 140, 145, 150, sont particulièrement indiquées et peuvent être avantageusement utilisées comme matière première des compositions concentrées liquides de xylitol selon l'invention.

Pour mettre en oeuvre le procédé selon l'invention lorsque l'on utilise des dextrines comme matières premières, celles-ci sont mises en solution dans l'eau à une matière sèche d'environ 20 % à 45 %, de préférence de 30 % à 40 % afin de subir la saccharification en glucose à l'aide d'au moins une enzyme saccharifiante telle que l'amyloglucosidase.

De préférence, et bien que cela ne soit pas nécessaire dans les cas où l'on utilise une dextrine assez transformée, on fait précéder cette action enzymatique de l'amyloglucosidase par l'action d'une alpha amylase de préférence thermorésistante.

L'action enzymatique de l'amyloglucosidase et éventuellement de l'alpha amylase sur la dextrine permet d'obtenir une fraction essentiellement composée de glucose et une autre essentiellement composée de polymères de glucose. Ces molécules polymériques ne seront pas transformées par les opérations microbiologiques et enzymatiques subséquentes mais seront transformées en polymères non réducteurs du glucose lors de l'hydrogénation catalytique finale. Ces polymères très viscosifiants joueront également le rôle d'anticristallisants du xylitol dans les compositions selon l'invention.

Quelle que soit la matière première retenue pour la mise en oeuvre du procédé selon l'invention, eaux mères de la cristallisation du dextrose, hydrolysat brut d'amidon acide-enzyme ou dextrine saccharifiée, celle-ci est généralement diluée à une matière sèche de 150 à 200 g/l puis elle est complémentée à l'aide de 2 à 4 g/l d'azote organique sous forme de corn steep ou d'extrait de levure et elle est additionnée de $KH_2PO_4$ à raison de 1 à 3 g/l et de $MgSO_4,7H_2O$ à raison de 1 à 2 g/l.

Le milieu de culture ainsi obtenu est introduit dans un fermenteur, puis il est stérilisé et inoculé à l'aide de 10 % environ d'une culture de 24 heures d'un microorganisme du genre Pichia.

La souche Pichia ohmeri n° ATCC 20.209 donne généralement de bons résultats.

La fermentation est poursuivie sous aération à une température voisine de 30°C durant 80 à 100 heures à un pH voisin de 4,5 avantageusement maintenu par de l'ammoniaque jusqu'à ce que la presque totalité du glucose soit transformée en D-arabitol.

Le contenu entier du fermenteur est alors stérilisé de manière à détruire la levure puis est à nouveau ensemencé par cette fois-ci, une préculture d'Acetobacter suboxydans.

La fermentation est alors poursuivie sous aération à une température de 20° à 40°C durant un temps généralement compris entre 24 et 48 heures, à un pH compris entre 4,0 et 6,0. A ce terme, pratiquement tout le D-arabitol a été oxydé en D-xylulose. Le moût ainsi obtenu est classiquement débarrassé des microorganismes par centrifugation ou filtration puis il est purifié de façon habituelle par des traitements de décoloration qui peuvent être effectués par du noir végétal et des traitements de déminéralisation sur résines cationiques et anioniques. Il est ensuite généralement concentré à une matière sèche de 40 %, optimale pour la réaction d'isomérisation enzymatique ultérieure.

On peut utiliser pour cette étape d'isomérisation enzymatique du xylulose en xylose, une glucose isomérase commerciale du type de celles mises en oeuvre pour la fabrication des sirops de maïs à haute teneur en fructose, à savoir par exemple celle qui est connue sous la marque SPEZYME$^R$ et qui est commercialisée par SUOMEN SOKERI ou celle qui peut être obtenue selon le brevet français n° 2.353.562 dont la demanderesse est titulaire.

De préférence, l'isomérisation est conduite à une température de 40° à 80°C et à un pH de préférence compris entre 6,0 et 8,5 généralement en présence d'un agent protecteur de l'enzyme tel que le bisulfite de soude et un sel de magnésium.

D'une façon générale, l'isomérisation est conduite durant un temps suffisant pour que 20 % à 75 % environ du D-xylulose soient convertis en D-xylose. Les taux les plus élevés d'isomérisation conduiront à des compositions contenant peu de D-arabitol tandis que des taux d'isomérisation plus faibles fourniront des compositions beaucoup plus riches en D-arabitol. Le sirop riche en D-xylose obtenu après cette étape d'isomérisation enzymatique et contenant alors généralement de 5 % à 35 % d'oligomères ou polymères de glucose, de 25 % à 75 % de xylulose et de 20 % à 70 % de xylose est alors purifié par déminéralisation puis est ensuite soumis à l'hydrogénation catalytique en présence de nickel de Raney ou de catalyseurs ou ruthénium.

De préférence, l'étape d'hydrogénation est effectuée avec un catalyseur ou nickel de Raney sous une pression d'hydrogène comprise entre 40 et 70 bars, à une température de 100° à 150°C et à une concentration de 20 % à 60 %.

L'hydrogénation est poursuivie jusqu'à ce que la teneur en sucres réducteurs du sirop hydrogéné soit inférieure à 2 %, de préférence inférieure à 1 % et plus préférentiellement encore inférieure à 0,5 %.

Le sirop hydrogéné ainsi obtenu est ensuite filtré pour enlever le catalyseur puis il est déminéralisé et avantageusement concentré pour fournir les compositions de l'invention.

## EXEMPLE 1 : Cas de l'utilisation d'hydrols.

### a) transformation du glucose en D-arabitol

Dans un fermenteur d'une capacité utile de 10m$^3$, on introduit :

- 8.000 litres d'un hydrol correspondant aux eaux mères de la cristallisation d'un premier jet de dextrose monohydrate, dilué de manière à obtenir une solution à 170 grammes/litre de matières sèches.
- 16 kilogrammes d'extrait de levure
- 8 kilogrammes de $KH_2PO_4$
- 8 kilogrammes de $MgSO_4,7H_2O$

L'analyse glucidique de l'hydrol montrait le spectre suivant :

| | |
|---|---|
| Glucose | 86,1 % |
| Maltose | 9,5 % |
| Maltotriose (DP3) | 2,0 % |
| Maltotétraose (DP4) | 0,5 % |
| DP5 | 0,3 % |
| DP6 | 0,4 % |
| DP7 | 0,5 % |
| DP8 à 20 | 0,4 % |
| DP > 20        nd env. | 0,3 % |

Après stérilisation du milieu de culture et refroidissement à 30°C, on a inoculé ce fermenteur à l'aide de 800 litres d'une préculture de Pichia ohmeri n° ATCC 20.209 telle que décrite dans le brevet français n° 2.009.331, préculture âgée de 24 heures.

L'aération a été poursuivie durant toute la durée de la transformation du glucose en D-arabitol, soit durant 90 heures avec un débit de 130 Nm$^3$/heure et le pH a été maintenu par addition d'ammoniaque, à une valeur de 4,5.

Au terme de cette première fermentation, l'analyse glucidique du moût de culture montrait l'analyse suivante :

| | |
|---|---|
| D-Arabitol | 78,8 % |
| Maltose | 14,5 % |
| DP3 | 3,2 % |
| DP4 | 0,8 % |
| DP5 | 0,5 % |
| DP6 | 0,5 % |
| DP7 | 0,8 % |
| DP8 à 20 | 0,5 % |
| DP > 20 | 0,4 % |

### b) transformation du D-arabitol en D-xylulose

Le contenu entier du fermenteur a été porté 20 minutes à 120°C puis après refroidissement à 30°C a été ensemencé avec 600 litres d'une préculture d'Acetobacter suboxydans. Après 24 heures de fermentation sous une aération de 1 volume/volume/minute, on a arrêté la fermentation, filtré les bactéries et les résidus de levure puis procédé à la décoloration sur charbon actif et à la déminéralisation sur résines échangeuses d'ions, cationique forte et anionique faible puis lit mixte cation fort-anion fort.

L'analyse glucidique du sirop obtenu était alors la suivante :

| Xylulose | 77,0 % |
|----------|--------|
| Maltose | 15,7 % |
| DP3 | 3,5 % |
| DP4 | 0,9 % |
| DP5 | 0,5 % |
| DP6 | 0,5 % |
| DP7 | 0,9 % |
| DP8 à 20 | 0,5 % |
| DP > 20 | 0,5 % |

c) isomérisation du D-xylulose en D-xylose

Ce sirop a été concentré à une matière sèche de 40 % puis il a été percolé à une température de 65°C sur une colonne de glucose isomérase immobilisée de marque SPEZYME$^R$ après avoir été ajusté à un pH de 7,7 par du carbonate de sodium et après addition de 0,4 g/l de bisulfite de sodium et de 1 g/l de chlorure de magnésium. Ce sirop a été percolé à un débit de 2 volumes de sirop par volume de colonne et par heure.

On a obtenu par cette réaction d'isomérisation un sirop de xylose de la composition suivante :

| Xylulose | 20,0 % |
|----------|--------|
| Xylose | 57,0 % |
| Maltose | 15,7 % |
| DP $\geq$ 3 | 7,3 % |

On constate donc que le maltose et les oligomères et polymères de glucose sont demeurés intransformés au cours de toutes ces étapes et qu'ils n'ont nullement entravé les transformations microbiologiques et enzymatiques du glucose en D-xylose.

d) hydrogénation

Ce sirop riche en xylose a été déminéralisé sur un lit mixte de résines puis il a été hydrogéné sous une pression d'hydrogène de 50 bars en présence de nickel de Raney et à une température de 120°C. Après les 3 heures d'hydrogénation, ce sirop présentait une teneur en sucres réducteurs résiduels inférieure à 0,1 %.

Ce sirop a été déminéralisé puis concentré à différentes matières sèches. Sa composition glucidique sur base sèche était alors la suivante :

| Xylitol | 67,0 % |
|---------|--------|
| Arabitol | 10,0 % |

(suite)

| Maltitol | 16,0 % |
|---|---|
| Oligo et Polysaccharides non réducteurs de glucose de DP ≥ 3 | 7,0 % |

EXEMPLE 2 : Cas de l'utilisation d'un hydrolysat d'amidon acide/enzyme

Un lait d'amidon d'une teneur en matières sèches de 36 % a été additionnée de 4 g/l d'acide chlorhydrique technique.

Il a été pompé dans l'espace annulaire d'un convertisseur constitué de trois tubes coaxiaux dont le plus grand et le plus petit sont chauffés par de la vapeur à 150°C. Le temps de séjour dans le convertisseur a été ajusté de manière à ce que le sirop de glucose obtenu montrait un DE de 37.

Après refroidissement à 55°C et rectification du pH à 5,0 on a ajouté 0,8 ‰ en poids/poids sec d'amyloglucosidase de la marque DEXTROZYME[R] de NOVO par kg d'amidon.

On a laissé la saccharification se poursuivre durant 80 heures au terme desquelles on a obtenu un hydrolysat qui après purification, montrait le spectre glucidique suivant :

| Glucose | 88,3 % |
|---|---|
| Maltose | 3,8 % |
| DP3 | 2,0 % |
| DP4 | 1,0 % |
| DP5 | 1,0 % |
| DP6 | 0,8 % |
| DP7 | 0,7 % |
| DP8 à 20 | 2,3 % |
| DP > 20 | 0,1 % |

Cet hydrolysat a été dilué à une matière sèche de 170 g/l et a été mis en oeuvre dans toutes les opérations décrites à l'exemple 1.

Au terme de toutes ces opérations, on a obtenu un sirop de xylitol de la composition suivante :

| | Xylitol | 70,3 % |
|---|---|---|
| | D-arabitol | 10,1 % |
| | Maltitol | 6,3 % |
| | DP ≥ 3 | 13,3 % |
| dont | DP3 | 3,4 % |
| | DP4 | 1,7 % |
| | DP5 | 1,5 % |
| | DP6 | 1,4 % |
| | DP7 | 1,4 % |
| | DP8 à 20 | 3,6 % |
| | DP > 20 | 0,2 % |

EXEMPLE 3 : Transformation d'une dextrine

Une dextrine blanche commercialisée par la société demanderesse sous la dénomination TACKIDEX[R] 135 a été dissoute dans de l'eau chaude à une matière sèche de 30 %. Le pH de cette solution a été ajusté à 5,5 et cette solution de dextrine a été thermostatée à 55°C.

On y a alors ajouté 0,8 ‰ en poids/poids sec d'amyloglucosidase de la marque DEXTROZYME[R] de NOVO par kg de dextrine.

On a laissé la saccharification se poursuivre durant 75 heures au terme desquelles on a obtenu un hydrolysat qui montrait le spectre glucidique suivant :

| | |
|---|---|
| Glucose | 87,7 % |
| DP2 | 2,2 % |
| DP3 | 0,4 % |
| DP4 | 2,9 % |
| DP5 | 1,7 % |
| DP6 à 20 | 4,0 % |
| DP > 20 | 1,1 % |

Cet hydrolysat a été dilué à une matière sèche de 170 g/l et a été mis en oeuvre dans toutes les opérations décrites à l'exemple 1.

L'isomérisation enzymatique a toutefois été conduite à un débit de quatre volumes de sirop par volume de colonne et par heure, fournissant un sirop de la composition suivante :

| | |
|---|---|
| Xylulose | 41,8 % |
| Xylose | 31,8 % |
| DP2 | 3,7 % |
| DP ≥ 3 | 17,0 % |

Au terme de toutes ces opérations, on a obtenu un sirop de xylitol de la composition suivante :

| | |
|---|---|
| Xylitol | 55,5 % |
| D-arabitol | 23,8 % |
| DP2 | 3,7 % |
| DP3 | 0,7 % |
| DP4 | 4,9 % |
| DP5 | 2,9 % |
| DP6 à 20 | 6,7 % |
| DP > 20 | 1,8 % |
| soit DP ≥ 3 | 17,0 % |

EXEMPLE 4 : Fabrication par mélanges

Un sirop de xylitol peu visqueux et aisément cristallisable, obtenu par fermentation de dextrose pur selon le brevet européen n° 421.882 et suivant les modalités décrites dans l'exemple 1, de la composition suivante :

| Xylitol | 87 % |
|---|---|
| D-arabitol | 12 % |
| Divers | 1 % |

a été mélangé avec une dextrine TACKIDEX[R] 135 hydrogénée de façon à obtenir les compositions a et b suivantes :

| Composition a | Xylitol | 54,4 % |
|---|---|---|
| | D-arabitol | 7,5 % |
| | DP ≥ 3 | 37,5 % |
| Composition b | Xylitol | 79,0 % |
| | D-arabitol | 10,9 % |
| | DP ≥ 3 | 9,0 % |

Ces compositions ont été concentrées à différentes matières sèches.

EXEMPLE 5 : Comparatif

On a préparé des solutions de xylitol pur (c), des solutions de xylitol à 70 % de richesse en xylitol et 30 % de richesse en maltitol (d) et des solutions à 70 % de richesse en xylitol et 30 % de richesse en D-arabitol (e). Toutes ces solutions ont été concentrées à des matières sèches différenteS.

EXEMPLE 6 : Stabilité des compositions de l'invention

Les compositions liquides de xylitol de tous les exemples 1 à 5 ont été concentrées de manière à obtenir des teneurs en xylitol de 185 grammes de xylitol pour 100 grammes d'eau soit :

- pour les compositions de l'exemple 1, une concentration totale de matières sèches de 73,4 % en poids pour poids,
- pour celles de l'exemple 2, de 72,5 %,
- pour celles de l'exemple 3, de 76,9 %,
- pour la composition a de l'exemple 4, de 77,3 %,
- pour la composition b de l'exemple 4, de 70,1 %,
- pour la solution c de l'exemple 5, de 64,9 %,
- pour les compositions d et e de l'exemple 5, de 72,5 %.

Pour toutes ces compositions, la teneur en xylitol s'établissait à 185 g de xylitol pour 100 g d'eau.
A 20°C, après six semaines, seules les compositions c, d et e de l'exemple 5 avaient cristallisé.
A 30°C, au terme du même délai, aucune composition n'a cristallisé.
A 16°C, après également six semaines toutes les compositions des exemples 1 - 2 - 3 et a et b de l'exemple 4 ne contenaient que des microcristaux à peine palpables contrairement aux compositions c, d et e de l'exemple 5 qui montraient une formation abondante de gros cristaux de xylitol.
Les mêmes compositions liquides des exemples 1 à 5 ont été concentrées de manière à obtenir des teneurs en xylitol de 310 g de xylitol pour 100 grammes d'eau soit :

- pour les compositions de l'exemple 1, une concentration totale de matières sèches de 82,3 % en poids pour poids,

- pour celles de l'exemple 2, de 81,5 %,
- pour celles de l'exemple 3, de 84,8 %,
- pour la composition a de l'exemple 4, de 85 %,
- pour la composition b de l'exemple 4, de 79,7 %,
- pour la solution c de l'exemple 5, de 75,6 %,
- pour les compositions d et e de l'exemple 5, de 81,6 %.

Pour toutes ces compositions, la teneur en xylitol s'établissait à 310 g de xylitol pour 100 g d'eau.

A 40°C, après six semaines, seules les compositions c, d et e de l'exemple 5 avaient cristallisé.

A 45°C, au terme du même délai, aucune composition n'a cristallisé.

A 37°C, après également six semaines, toutes les compositions des exemples 1 - 2 - 3 et a et b de l'exemple 4 ne contenaient que des microcristaux à peine palpables contrairement aux compositions c, d et e de l'exemple 5 qui montraient une formation abondante de cristaux de xylitol.

Ces exemples démontrent que lorsque la teneur en xylitol, exprimée en grammes pour 100 g d'eau des compositions selon l'invention se trouve comprise dans l'intervalle défini par les équations :

$$\frac{83,2x + 9130}{83 - x} \text{ et } \frac{91,5x + 10050}{83 - x}$$

x representant la température en degrés centigrades, qu'alors celles-ci demeurent incristallisables bien que le xylitol y soit en sursaturation.

De même, lorsque la teneur en xylitol des compositions selon l'invention se trouve comprise dans l'intervalle défini par les équations :

$$\frac{91,5x + 10050}{83 - x} \text{ et } \frac{100x + 11000}{83 - x}$$

Celles-ci présentent alors des caractéristiques de cristallisation retardée.

EXEMPLE 7 : Viscosité des compositions selon l'invention

On a comparé les temps nécessaires à l'écoulement de 50 ml de compositions conformes à l'invention à ceux de sirops de l'art antérieur. Ces temps d'écoulement sont directement proportionnels aux viscosités de compositions ou des sirops. En d'autres termes, plus ces temps sont élevés plus les viscosités sont grandes.

Pour cela, toutes les compositions des exemples 1 à 5 ont été portées à une concentration de 70 % de matières sèches totales en poids pour poids soit à une valeur où le xylitol est en deça de sa limite de solubilité (sauf pour la composition c de l'exemple 5). 50 ml de chacune des compositions sont alors placés dans une burette de 1 cm de diamètre. On a mesuré à l'aide d'un chronomètre les temps d'écoulement.

On a obtenu à 23°C les valeurs suivantes :

- composition de l'exemple 1 : 811 secondes
- composition de l'exemple 2 : 798 secondes
- composition de l'exemple 3 : 823 secondes
- composition a de l'exemple 4 : 1709 secondes
- composition b de l'exemple 4 : 529 secondes
- composition c de l'exemple 5 : 346 secondes
- composition d de l'exemple 5 : 710 secondes
- composition e de l'exemple 5 : 340 secondes
- eau pure : 48 secondes

Ces mesures démontrent que les compositions liquides selon l'invention sont plus visqueuses et ont plus de corps que les sirops de l'art antérieur.

**Revendications**

1. Compositions liquides visqueuses de xylitol caractérisées par le fait qu'elles contiennent :

- de 51 % à 80 % de xylitol,
- de 0,1 % à 44 % de D-arabitol,
- de 5 % à 48,9 % d'oligomères ou de polymères non réducteurs du glucose, ces pourcentages étant exprimés sur la base sèche des compositions.

2. Compositions liquides visqueuses de xylitol selon la revendication 1 présentant des caractéristiques de cristallisation retardée ou qui sont incristallisables à températures données, caractérisées par le fait que leur teneur en eau est ajustée de manière à ce que leur teneur en xylitol, exprimée en grammes de xylitol pour 100 grammes d'eau soit comprise entre les valeurs définies par les équations :

$$\frac{83,2x + 9130}{83 - x} \text{ et } \frac{100x + 11000}{83 - x}$$

x réprésentant la température en degrés centigrades des compositions.

3. Compositions liquides visqueuses de xylitol selon la revendication 1, incristallisables à températures données, caractérisées par le fait que leur teneur en eau est ajustée de manière à ce que leur teneur en xylitol, exprimée en grammes de xylitol pour 100 grammes d'eau soit comprise entre les valeurs définies par les équations :

$$\frac{83,2x + 9130}{83 - x} \text{ et } \frac{91,5x + 10050}{83 - x}$$

x représentant la température en degrés centigrades des compositions.

4. Compositions liquides visqueuses de xylitol selon les revendications 1 et 2, présentant des caractéristiques de cristallisation retardée à 20°C, caractérisée par le fait que leur teneur en eau est ajustée de manière à ce que leur teneur en xylitol, exprimée en grammes de xylitol pour 100 grammes d'eau soit comprise entre 188 g et 206 g.

5. Compositions liquides visqueuses de xylitol selon les revendications 1 et 3, incristallisables à 20°C, caractérisées par le fait que leur teneur en eau est ajustée de manière à ce que leur teneur en xylitol, exprimée en grammes de xylitol pour 100 grammes d'eau, soit comprise entre 171 g et 188 g.

6. Procédé de fabrication de compositions concentrées liquides de xylitol selon les revendications 1 à 5 consistant à procéder aux transformations microbiologiques du glucose en D-arabitol puis D-xylulose, à l'isomérisation enzymatique du D-xylulose en un mélange de D-xylose et de D-xylulose puis à l'hydrogénation catalytique de ce mélange, caractérisé en ce que les transformations microbiologiques, l'isomérisation et l'hydrogénation ont lieu en présence d'oligomères et/ou de polymères réducteurs du glucose.

7. Procédé selon la revendication 6 caractérisé par le fait que les oligomères et/ou polymères réducteurs du glucose sont apportés par mise en oeuvre d'eaux mères de la cristallisation du dextrose.

8. Procédé selon la revendication 6 caractérisé par le fait que les oligomères et/ou polymères réducteurs du glucose sont apportés par la mise en oeuvre d'un hydrolysat d'amidon obtenu par liquéfaction acide puis saccharification enzymatique.

9. Procédé selon la revendication 6 caractérisé par le fait que les oligomères et/ou polymères réducteurs du glucose sont apportés par la mise en oeuvre d'une dextrine ayant subi une saccharification enzymatique.

**Claims**

1. Viscous liquid compositions of xylitol characterized by the fact that they contain:

- from 51% to 80% of xylitol,
- from 0.1% to 44% of D-arabitol,
- from 5% to 48.9% of non-reducing oligomers or polymers of glucose, these percentages being expressed on a dry weight basis of the compositions.

**EP 0 661 930 B1**

2. Viscous liquid compositions of xylitol according to Claim 1, having retarded crystallization characteristics or which are noncrystallizable at given temperatures, characterized by the fact that their water content is adjusted so that their xylitol content, expressed in grams of xylitol per 100 grams of water, is between the values defined by the equations:

$$\frac{83.2x + 9130}{83 - x} \text{ and } \frac{100x + 11000}{83 - x}$$

x representing the temperature in degrees centigrade of the compositions.

3. Viscous liquid compositions of xylitol according to Claim 1, which are noncrystallizable at given temperatures, characterized by the fact that their water content is adjusted so that their xylitol content, expressed in grams of xylitol per 100 grams of water, is between the values defined by the equations:

$$\frac{83.2x + 9130}{83 - x} \text{ and } \frac{91.5x + 10050}{83 - x}$$

x representing the temperature in degrees centigrade of the compositions.

4. Viscous liquid compositions of xylitol according to Claims 1 and 2, having retarded crystallization characteristics at 20°C, characterized by the fact that their water content is adjusted so that their xylitol content, expressed in grams of xylitol per 100 grams of water, is between 188 g and 206 g.

5. Viscous liquid compositions of xylitol according to Claims 1 and 3, which are noncrystallizable at 20°C, characterized by the fact that their water content is adjusted so that their xylitol content, expressed in grams of xylitol per 100 grams of water, is between 171 g and 188 g.

6. Process for the manufacture of concentrated liquid compositions of xylitol according to Claims 1 to 5, consisting in carrying out the microbiological conversions of glucose to D-arabitol and then D-xylulose, in the enzymatic isomerization of D-xylulose to a mixture of D-xylose and D-xylulose and then in the catalytic hydrogenation of this mixture, characterized in that the microbiological conversions, the isomerization and the hydrogenation occur in the presence of reducing oligomers and/or polymers of glucose.

7. Process according to Claim 6, characterized by the fact that the reducing oligomers and/or polymers of glucose are provided by the use of mother liquors from the crystallization of dextrose.

8. Process according to Claim 6, characterized by the fact that the reducing oligomers and/or polymers of glucose are provided by the use of a starch hydrolysate obtained by acid liquefaction followed by enzymatic saccharification.

9. Process according to Claim 6, characterized by the fact that the reducing oligomers and/or polymers of glucose are provided by the use of a dextrin having undergone an enzymatic saccharification.

**Patentansprüche**

1. Flüssige, viskose Zusammensetzungen von Xylitol, dadurch gekennzeichnet, daß sie enthalten:

   - 51 % bis 80 % Xylitol,
   - 0,1 % bis 44 % D-Arabitol,
   - 5 % bis 48,9 % nicht reduzierende Oligomere oder Polymere der Glucose,
     wobei diese Prozentsätze in Trockenbasis der Zusammensetzungen ausgedrückt sind.

2. Flüssige, viskose Zusammensetzungen von Xylitol nach Anspruch 1, die Charakteristiken von verzögerter Kristallisation aufweisen oder die bei vorgegebenen Temperaturen nicht kristallisierbar sind, dadurch gekennzeichnet, daß ihr Gehalt an Wasser in der Weise eingestellt ist, daß ihr Gehalt an Xylitol, ausgedrückt in Gramm Xylitol pro 100 g Wasser, zwischen den durch die folgenden Gleichungen definierten Werten liegt:

$$\frac{83{,}2x + 9130}{83 - x} \quad \text{und} \quad \frac{100x + 11000}{83 - x}$$

worin x die Temperatur der Zusammensetzungen in Grad Celsius darstellt.

3. Flüssige, viskose Zusammensetzungen von Xylitol nach Anspruch 1, die bei vorgegebenen Temperaturen nicht kristallisierbar sind, dadurch gekennzeichnet, daß ihr Gehalt an Wasser in der Weise eingestellt ist, daß ihr Gehalt an Xylitol, ausgedrückt in Gramm Xylitol pro 100 g Wasser, zwischen den durch die folgenden Gleichungen definierten Werten liegt:

$$\frac{83{,}2x + 9130}{83 - x} \quad \text{und} \quad \frac{91{,}5x + 10050}{83 - x}$$

worin x die Temperatur der Zusammensetzungen in Grad Celsius darstellt.

4. Flüssige, viskose Zusammensetzungen von Xylitol nach Anspruch 1 und 2, die bei 20 °C Charakteristiken von verzögerter Kristallisation aufweisen, dadurch gekennzeichnet, daß ihr Gehalt an Wasser in der Weise eingestellt ist, daß ihr Gehalt an Xylitol, ausgedrückt in Gramm Xylitol pro 100 g Wasser, zwischen 188 g und 206 g liegt.

5. Flüssige, viskose Zusammensetzungen von Xylitol nach Anspruch 1 und 3, die bei 20 °C nicht kristallisierbar sind, dadurch gekennzeichnet, daß ihr Gehalt an Wasser in der Weise eingestellt ist, daß ihr Gehalt an Xylitol, ausgedrückt in Gramm Xylitol pro 100 g Wasser, zwischen 171 g und 188 g liegt.

6. Verfahren zur Herstellung von flüssigen, konzentrierten Zusammensetzungen von Xylitol nach Anspruch 1 bis 5, das darin besteht, mikrobiologische Umwandlungen von Glucose zu D-Arabitol und anschließend zu D-Xylulose bei der enzymatischen Isomerisierung von D-Xylulose zu einer Mischung von D-Xylose und D-Xylulose und anschließend die katalytische Hydrierung dieser Mischung durchzuführen, dadurch gekennzeichnet, daß die mikrobiologischen Umwandlungen, die Isomerisierung und die Hydrierung in Anwesenheit von reduzierenden Oligomeren und/oder Polymeren der Glucose stattfinden.

7. Verfahren nach Anspruch 6, dadurch gekennzeichnet, daß die reduzierenden Oligomere und/oder Polymere der Glucose durch Verwendung von Mutterlaugen aus der Kristallisation von Dextrose eingebracht werden.

8. Verfahren nach Anspruch 6, dadurch gekennzeichnet, daß die reduzierenden Oligomere und/oder Polymere der Glucose durch Verwendung eines Stärkehydrolysates eingebracht werden, das durch saure Verflüssigung und nachfolgende enzymatische Verzuckerung erhalten wurde.

9. Verfahren nach Anspruch 6, dadurch gekennzeichnet, daß die reduzierenden Oligomere und/oder Polymere der Glucose durch Verwendung eines Dextrins eingebracht werden; das einer enzymatischen Verzuckerung unterzogen wurde.